(19) Europäisches Patentamt
European Patent Office
Office européen des brevets

(11) Veröffentlichungsnummer: **0 032 366**
**B1**

# (12) EUROPÄISCHE PATENTSCHRIFT

(45) Veröffentlichungstag der Patentschrift: **07.09.83**

(51) Int. Cl.³: **C 07 D 233/96**, A 01 N 43/50

(21) Anmeldenummer: **81100004.1**

(22) Anmeldetag: **02.01.81**

## (54) Verwendung von 4-Thioparabansäure-Derivaten als herbizide Wirkstoffe.

(30) Priorität: **04.01.80 DE 3000130**

(43) Veröffentlichungstag der Anmeldung: **22.07.81 Patentblatt 81/29**

(45) Bekanntmachung des Hinweises auf die Patenterteilung: **07.09.83 Patentblatt 83/36**

(84) Benannte Vertragsstaaten: **AT BE CH DE FR GB IT LI NL**

(56) Entgegenhaltungen: **US-A-3 843 677**
**US-A-3 854 925**

(73) Patentinhaber: **Consortium für elektrochemische Industrie GmbH, Zielstattstrasse 20, D-8000 München 70 (DE)**

(72) Erfinder: **Kinzel, Peter, Ing. grad., Jägerkampstrasse 21a, D-8152 Westerham/Feldkirchen (DE)**
Erfinder: **Mack, Wilhelm, Dr. Dipl.-Chem., Unghausen 24, D-8263 Burghausen (DE)**

EP 0 032 366 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verwendung von 4-Thioparabansäure-Derivaten als herbizide Wirkstoffe

Die Erfindung betrifft die Verwendung von Verbindungen aus der Reihe der 2-Oxo-4-thiono-5-imino-imidazolidine, im folgenden als 4-Thioparabansäure-Derivate bezeichnet, als herbizide Wirkstoffe.

Aufgabe der Erfindung war es, 4-Thioparabansäure-Derivate aufzuzeigen, die als herbizide Wirkstoffe gegen unerwünschte Pflanzen in Kulturpflanzungen einsetzbar sind und die ein für eine derartige Anwendung zu forderndes Toleranzverhalten gegenüber Nutzpflanzen aufweisen.

Gegenstand der Erfindung ist demnach die Verwendung einer oder mehrerer Verbindungen der allgemeinen Formel I

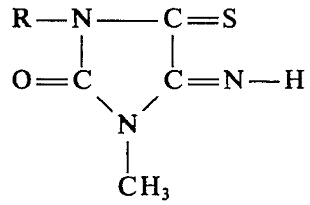

(I)

als herbizide Wirkstoffe,
in der R entweder die Naphthylgruppe oder eine einfach oder mehrfach substituierte Phenylgruppe bedeutet, wobei als Substituenten in Betracht kommen:
Methyl, Ethyl, Propyl, Isopropyl, Methoxy, Ethoxy, Propoxy, Methylmercapto, Ethylmercapto, Propylmercapto, Methoxycarbonyl, Ethoxycarbonyl, Propoxycarbonyl, Methylsulfonato, Ethylsulfonato, Trifluormethyl, Halogen, die Nitro- und die Cyanogruppe.

Die erfindungsgemäß zu verwendenden Wirkstoffe können zur selektiven Bekämpfung von Schadpflanzen bei entsprechenden Aufwandmengen ohne schädigende Einflüsse auf die Nutzpflanzen in Kulturen des Acker- und Gartenbaus eingesetzt werden. Ihre herbizide Wirksamkeit erstreckt sich u. a. auf stark ertragsmindernde Ungräserarten und auf wichtige dikotyle Unkräuter. Aus der Gruppe der Süßgräser seien beispielhaft genannt die Lolium-Arten Poa annua, Alopecurus myosuroides, Avena fatua, einige Hirsearten, wie Digitaria sanguinalis und Echinochloa crus galli, sowie aus der Gruppe der Sauergräser die vor allem im Reisanbau bedeutsame Cyperus iria. Weitere Beispiele für dikotyle Unkräuter sind Kreuzblütler, wie Sinapis arvensis, Capsella bursa-pastoris, Thlaspi arvensis, Raphanus raphanistrum, u. a., Korbblütler, wie Centaurea cyanus, Matricaria inodora, Chrysanthemum segetum, Galinsoga parviflora, Senecio vulgaris, u. a., Knötericharten, wie Polygonum persicaria, Lippenblütler, wie Lamium purpureum sowie Chenopodium album, Stellaria media, Galium aparine, Amaranthus retroflexus, u. a.

Die erfindungsgemäß zu verwendenden Wirkstoffe bewähren sich als herbizide Mittel in Kulturpflanzungen, wie Sommergerste, Winterweizen, Reis, Baumwolle, Sojabohne, Kartoffeln, Kulturhirse — insbesondere Sorghum-Hirse, Mais, Leguminosen, wie Luzerne, Bohnen, Erbsen sowie in Karottenpflanzungen.

Die Darstellung der erfindungsgemäß zu verwendenden Verbindungen erfolgt durch Umsetzen von organischen Thio-oxanil-säure-nitrilen mit Methylisocyanat.

Das Verfahren zur Herstellung der Verbindungen der allgemeinen Formel

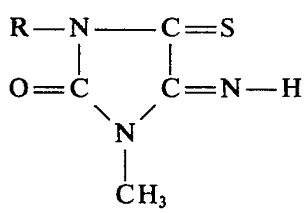

wobei R die oben definierte Bedeutung hat, besteht darin, daß

a) ein Thio-oxanil-säure-nitril der allgemeinen Formel II

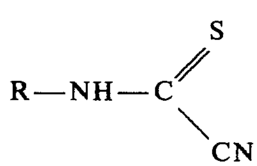

(II)

wobei R die oben definierte Bedeutung hat, mit Methylisocyanat umgesetzt wird, und

b) ggf. die Cyclisierungsreaktion fördernde Lösungsmittel während oder nach der unter a) beschriebenen Reaktion eingesetzt werden.

Der Verlauf der dem Verfahren zugrundeliegenden chemischen Reaktion hängt ab von der Reaktionstemperatur, dem Reaktionsmedium sowie von der Reinheit der Ausgangssubstanzen (Thio-oxanil-säure-nitril bzw. Isocyanat), insbesondere vom Chlorwasserstoffgehalt, der sowohl als Verunreinigung aus dem Herstellungsprozeß, wie auch von einer kontrollierten, reproduzierbaren Zudosierung stammen kann.

Chlorwasserstoff besitzt, unter der Maßgabe, daß kein basisches Reaktionsmedium, wie beispielsweise Pyridin, verwendet wird, eine reaktionsverzögernde Wirkung. Ein Chlorwasserstoffgehalt bis zu 1000 ppm, bezogen auf die chlorwasserstoffhaltige Komponente, kann jedoch im allgemeinen toleriert werden. Es ist andererseits oft zweckmäßig, dem umzusetzenden Methylisocyanat 50 bis 1000 ppm, vorzugsweise 150 bis 250 ppm, HCl, jeweils bezogen auf die Gewichtsmenge Isocyanat, zuzusetzen, um eine weitergehende Reaktion am Iminstickstoff (an der 5-Stellung) mit überschüssigem Isocyanat zu unterdrücken.

Die Umsetzungen werden vorteilhafterweise in einem Temperaturbereich von 0 bis 100°C, vorzugsweise bei Temperaturen um 20°C, durchgeführt.

Pyridin bzw. Pyridinbasen, wie beispielsweise die Piccoline, als Reaktionsmedium sichern im allgemeinen einen schnellen und glatten Verlauf der Umsetzungen.

Oftmals kann jedoch das umzusetzende Isocyanat selbst als Reaktionsmedium eingesetzt werden, wenn das Isocyanat ein gegenüber dem Thio-oxanil-säure-nitril ausreichendes Lösevermögen aufweist.

Wird in aprotischen, wasserfreien Lösungsmitteln, wie Benzol, Toluol, Tetrahydrofuran u. a. gearbeitet, sowie bei Umsetzungen ohne Lösungsmittel im unteren Temperaturbereich, bleibt die Cyclisierungsreaktion im allgemeinen aus, es entstehen Verbindungen der allgemeinen Formel III,

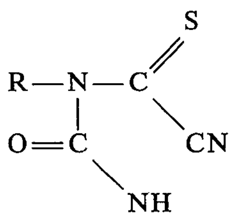

(III)

die beim Lösen oder durch Umkristallisieren in, die Cyclisierungsreaktion fördernden Lösungsmitteln, wie hydroxylhaltigen Lösungsmitteln, z. B. Ethanol, wasserhaltigem Tetrahydrofuran aber auch in basischen Lösungsmitteln, wie z. B. Pyridin oder Pyridinbasen, spontan in das entsprechende cyclische Produkt übergeführt werden.

Die als Ausgangssubstanzen einzusetzenden Thio-oxanil-säure-nitrile sind aus den entsprechenden, ggf. am Phenylrest substituierten Senfölen zugänglich. Ihre Herstellung erfolgt nach bzw. analog nach der Vorschrift von A. Reißert und K. Brüggemann in Ber. dtsch. Chem. Ges. 57, 981 (1924):

Phenylsenföl, bzw. substituiertes Phenylsenföl wird in alkoholischer Lösung mit einer wäßrigen Lösung von KCN verrührt. Nach beendeter Reaktion (im allgemeinen nach ca. 3 Stunden) wird auf das doppelte Volumen verdünnt und mit Salzsäure gefällt. Das anfallende Rohprodukt kann, falls gewünscht, beispielsweise aus einem Benzol/Cyclohexan-Gemisch umkristallisiert werden. Dieses Verfahren ist allgemein anwendbar. Es sind damit auch solche erfindungsgemäß einzusetzende Thio-oxanil-säure-nitrile zugänglich, die noch nicht in der Literatur beschrieben sind.

Der Phenylrest der erfindungsgemäß einzusetzenden Thio-oxanil-säure-nitrile kann in o-, m- oder p-Stellung einfach oder mehrfach substituiert sein.

Beispiele für Substituenten am Phenylrest sind Methyl, Ethyl, Propyl, Isopropyl, Methoxy, Ethoxy, Propoxy, Methylmercapto, Ethylmercapto, Propylmercapto, Methoxycarbonyl, Ethoxycarbonyl, Propoxycarbonyl, Methylsulfonato, Ethylsulfonato, Trifluormethyl, Halogen, die Nitro- und die Cyanogruppe.

Im folgenden werden Beispiele für erfindungsgemäß zu verwendende Verbindungen der allgemeinen Formel I

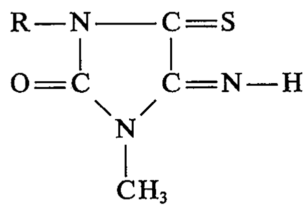

(I)

genannt:

1-Methyl-3-p-chlorphenyl-4-thioparabansäure-5-imid
1-Methyl-3-o-chlorphenyl-4-thioparabansäure-5-imid
1-Methyl-3-m-chlorphenyl-4-thioparabansäure-5-imid
1-Methyl-3-o,p-dichlorphenyl-4-thioparabansäure-5-imid
1-Methyl-3-m,p-dichlorphenyl-4-thioparabansäure-5-imid
1-Methyl-3-p-fluorphenyl-4-thioparabansäure-5-imid
1-Methyl-3-(p-trifluormethyl-phenyl)-4-thioparabansäure-5-imid
1-Methyl-3-p-nitrophenyl-4-thioparabansäure-5-imid
1-Methyl-3-o,p-dinitrophenyl-4-thioparabansäure-5-imid
1-Methyl-3-p-methylphenyl-4-thioparabansäure-5-imid
1-Methyl-3-m-methylphenyl-4-thioparabansäure-5-imid
1-Methyl-3-o-methylphenyl-4-thioparabansäure-5-imid
1-Methyl-3-m,p-dimethylphenyl-4-thioparabansäure-5-imid
1-Methyl-3-o,p-dimethylphenyl-4-thioparabansäure-5-imid
1-Methyl-3-o,o,p-trimethylphenyl-4-thioparabansäure-5-imid
1-Methyl-3-p-ethylphenyl-4-thioparabansäure-5-imid
1-Methyl-3-m-ethylphenyl-4-thioparabansäure-5-imid
1-Methyl-3-o,p-diethylphenyl-4-thioparabansäure-5-imid
1-Methyl-3-p-propylphenyl-4-thioparabansäure-5-imid
1-Methyl-3-p-isopropylphenyl-4-thioparabansäure-5-imid
1-Methyl-3-p-methoxyphenyl-4-thioparabansäure-5-imid
1-Methyl-3-p-ethoxyphenyl-4-thioparabansäure-5-imid
1-Methyl-3-p-propoxyphenyl-4-thioparabansäure-5-imid
1-Methyl-3-p-methylmercaptophenyl-4-thioparabansäure-5-imid
1-Methyl-3-p-ethylmercaptophenyl-4-thioparabansäure-5-imid
1-Methyl-3-p-propylmercaptophenyl-4-thioparabansäure-5-imid
1-Methyl-3-p-cyanophenyl-4-thioparabansäure-5-imid
1-Methyl-3-m-cyanophenyl-4-thioparabansäure-5-imid
1-Methyl-3-p-methoxycarbonylphenyl-4-thioparabansäure-5-imid
1-Methyl-3-p-ethoxycarbonylphenyl-4-thioparabansäure-5-imid
1-Methyl-3-p-propoxycarbonylphenyl-4-thioparabansäure-5-imid
1-Methyl-3-p-methylsulfonatophenyl-4-thioparabansäure-5-imid
1-Methyl-3-p-ethylsulfonatophenyl-4-thioparabansäure-5-imid.

Die erfindungsgemäß zu verwendenden Wirkstoffe können allein oder im Gemisch ausgebracht werden. Im allgemeinen werden sie jedoch als Mischungen mit festen oder flüssigen Verdünnungsmitteln oder als Lösungen verwendet, mit Wirkstoffgehalten von 0,01 bis 95 Gew.-%.

Die Mischungen bzw. Lösungen werden im allgemeinen als Emulsionskonzentrate, Pasten, Spritzpulver, Granulate oder Mikrokapseln hergestellt.

Emulsionskonzentrate und Pasten enthalten im allgemeinen 10 bis 90 Gew.-%, vorzugsweise 15 bis 50 Gew.-%, Wirkstoff, 2 bis 25 Gew.-% Dispergierhilfsstoffe und organische Lösungsmittel und/oder Wasser.

Spritzpulver enthalten meistens 10 bis 80 Gew.-%, vorzugsweise 15 bis 70 Gew.-%, Wirkstoff, 1 bis 10 Gew.-% Dispergierhilfsstoffe und 10 bis 89 Gew.-% inerte Bestandteile.

Granulate und Mikrokapseln enthalten neben inerten Bestandteilen, Bindemitteln und/oder Überzugsstoffen 1 bis 10 Gew.-%, vorzugsweise 5 bis 10 Gew.-%, Wirkstoff.

Erfindungsgemäß angewandt werden:

Als Dispergierhilfsstoffe z. B. Alkyl- und Arylsulfonate, Methylcellulose, polymere Sulfonsäuren und deren Salze, Polyalkohole, Fettsäureester, Fettalkoholäther, Fettamine;
als organische Lösungsmittel z. B. Alkohole, wie Ethanol, Butanole, Dimethylformamid (DMF), Dimethylsulfoxid (DMSO), N-Methylpyrrolidon, Aromaten, wie Toluol und Xylole;
als inerte Bestandteile z. B. Kaolin, China-Clay, Talkum, Calciumcarbonat, hochdisperse Kieselsäure, Kieselgele, Kieselgur, Diatomenerde, Bims, Ziegelsplitt, Maisschrot, Verdickungsmittel, wie Stärke und Carboxymethylcellulose;
als Bindemittel z. B. Magnesiumsulfat, Gips, Gummiarabicum.

Beispielsweise werden erfindungsgemäße Wirkstoffe zur Verwendung als Herbizide wie folgt formuliert:

1. Emulsionskonzentrat:
   51,4 Gew.-% Wirkstoff
   30 Gew.-% Sillitin Z
   10 Gew.-% hochdisperse Kieselsäure (HDK)
   6 Gew.-% Ligninsulfonat (Zellpech)

2 Gew.-% Polypropylenglykol
0,6 Gew.-% Natriumoleyl-Methyltaurid

2. Spritzpulver:
20 Gew.-% Wirkstoff
44 Gew.-% Chinaclay
16 Gew.-% hochdisperse Kieselsäure
15 Gew.-% Ligninsulfonat (Zellpech)
5 Gew.-% Natrium-Alkylnaphthalinsulfonat-Formaldehydkondensat (»Atlox 4862«, eingetragenes Warenzeichen)

Im allgemeinen werden die Wirkstoffe in Aufwandmengen von 0,5 bis 8 kg/ha, vorzugsweise 2 bis 4 kg/ha, ausgebracht.
Die Anwendung als herbizider Wirkstoff kann sowohl auf die Pflanzen (Nachauflaufverfahren) als auch auf vegetationsfreie Bodenoberflächen (Vorauflauf- und Vorsaatverfahren) erfolgen. Der größere herbizide Effekt wird mit einer Bodenapplikation erzielt, vorzugsweise bei Anwendung vor der Aussaat der Kulturpflanzen mit nachfolgender flacher, mechanischer Einarbeitung des ausgebrachten herbiziden Mittels in die obersten Schichten der Bodenoberfläche.
Im folgenden werden Darstellungsmethoden der erfindungsgemäß zu verwendenden Verbindungen und ihre herbiziden Eigenschaften anhand von Beispielen erläutert:

Beispiel 1

Darstellung von 1-Methyl-3-phenyl-4-thioparabansäure-5-imid (III)

$R = C_6H_5$

1. Stufe:
20 g durch Umkristallisieren aus Benzol/Cyclohexan (1 : 1) gereinigtes Thio-oxanil-säure-nitril (I) werden bei 20°C in 30 ml frisch destilliertem, HCl-freiem Methylisocyanat gelöst. Beim Stehen über Nacht scheiden sich derbe, rotgelbe Kristalle ab. Nach 24 Stunden wird überschüssiges Methylisocyanat abdestilliert und der Rückstand aus Benzol umkristallisiert. Es wird in einer Ausbeute von 25 g (92% d. Th.) N-Methylcarbamoyl-thio-oxanil-säure-nitril (II) mit einem Schmelzpunkt 116°C (Zers.) erhalten.

2. Stufe:
20 g (II) werden in der eben ausreichenden Menge kochenden Ethanols gelöst. Beim Abkühlen werden in einer Ausbeute von 19 g (95% d. Th.) gelbe Kristalle von 1-Methyl-3-phenyl-4-thioparabansäure-5-imid (III) mit einem Schmelzpunkt von 132°C erhalten.

Beispiel 2

Herstellung von 1-Methyl-3-phenyl-4-thioparabansäure-5-imid (III)

$R = C_6H_5$

60 g rohes, nicht umkristallisiertes Thio-oxanil-säure-nitril (IA), hergestellt nach der Vorschrift von A. Reißert und K. Brüggemann in Ber. dtsch. chem. Ges. 57, 981 (1924) werden bei 20°C mit 90 ml Methylisocyanat übergossen, dem vorher 8 ml Chlorwasserstoffgas zugesetzt wurden. Nach 24stündigem Stehenlassen wird überschüssiges Methylisocyanat abgezogen und der Rückstand aus Ethanol umkristallisiert. In einer Ausbeute von 55,7 g wird das Titelprodukt (III) erhalten. Schmelzpunkt 129 bis 131°C.

Vergleichsbeispiel 1

10 g reines Thio-oxanil-säure-nitril (I) (analog Beispiel 1) werden in 15 ml Methylisocyanat gelöst, dem 0,45 ml Chlorwasserstoffgas zudosiert wurden. Nach 24stündigem Stehenlassen wird ein schwarzer, teeriger Rückstand erhalten. Ein Thioparabansäureprodukt konnte nicht isoliert werden.

## Beispiel 3

### Herstellung von 1-Methyl-3-o-chlorphenyl-4-thioparabansäure-5-imid (IV)

$R = o\text{-}ClC_6H_4$

5 g o-Chlor-thio-oxanil-säure-nitril werden mit 20 ml HCl-freiem Methylisocyanat übergossen und 24 Stunden bei 20° C stehengelassen. Anschließend wird überschüssiges Methylisocyanat abgezogen, schließlich wird der Rückstand aus Ethanol umkristallisiert. Es werden gelbe Kristalle von (IV) erhalten in einer Ausbeute von 3,35 g. Schmelzpunkt 129 bis 131° C.

## Beispiel 4

$R = o\text{-}ClC_6H_4$

Es wird analog Beispiel 3 gearbeitet, mit der Abwandlung, daß statt bei 20° C 2 Stunden lang in siedendem Methylisocyanat umgesetzt wird. Die Verfahrensweise führt zu dem gleichen, wie im Beispiel 3 beschriebenen Ergebnis.

## Beispiel 5

### Herstellung von 1-Methyl-3-p-fluorphenyl-4-thioparabansäure-5-imid (V)

$R = p\text{-}F - C_6H_4$

5 g p-Fluor-thio-oxanil-säure-nitril werden mit 20 ml Methylisocyanat bei 20° C umgesetzt. Die weitere Verfahrensweise erfolgt analog Beispiel 3. Es werden gelbe Kristalle von (V) in einer Ausbeute von 5,2 g erhalten. Schmelzpunkt 122 bis 124° C.

## Beispiel 6

### Herstellung von 1-Methyl-3-o-methoxyphenyl-4-thioparabansäure-5-imid (VI)

$R = o\text{-}CH_3OC_6H_4$

38,4 g o-Methoxy-thio-oxanil-säure-nitril werden in 60 ml Pyridin gelöst, mit 11,4 g Methylisocyanat versetzt und 24 Stunden bei 20° C stehengelassen. Anschließend wird das Pyridin abgezogen und der Rückstand schließlich aus Ethanol umkristallisiert. Es entsteht, in einer Ausbeute von 29 g (VI) mit einem Schmelzpunkt von 124 bis 127° C.

## Beispiel 7

### Herstellung von 1-Methyl-3-(3,4-dichlorphenyl)-4-thioparabansäure-5-imid (VII)

$R = o,p\text{-}Cl_2C_6H_3$

10 g 3,4-Dichlor-thio-oxanil-säure-nitril werden in 60 ml Pyridin gelöst und 2,5 g Methylisocyanat hinzugefügt. Nach 24stündigem Stehenlassen wird das Pyridin im Vakuum abgezogen und der Rückstand in wenig heißem Ethanol aufgenommen. Es fallen 8,1 g an rohem (VII) an. Nach erneutem Umkristallisieren aus 100 ml Ethanol verbleiben 5,8 g an gelben Kristallen von (VII). Schmelzpunkt 135 bis 137° C.

## Beispiel 8

### Herstellung von 1-Methyl-3-tolyl-4-thioparabansäure-5-imid (VIII)

$R = p\text{-}CH_3C_6H_4$

10 g p-Methyl-thio-oxanil-säure-nitril, in 20 ml Pyridin gelöst, werden unter Kühlung mit 3,25 g Methylisocyanat umgesetzt. Nach 24 Stunden bei 20° C wird eingedampft und der Rückstand aus 50 ml

Ethanol umkristallisiert. Es werden gelbe Kristalle von (VIII) in einer Ausbeute von 7,9 g erhalten. Schmelzpunkt 135 bis 137° C.

## Beispiel 9

### Herbizide Wirksamkeit von 1-Methyl-3-phenyl-4-thioparabansäure-5-imid (III)

Die herbizide Wirksamkeit der erfindungsgemäßen Verbindung bei einmaliger Anwendung wurde in Gewächshausversuchen getestet. Im dargestellten Test wurden zunächst die Samenkörner der Kulturpflanzen und Unkräuter in mineralischem, humusarmem Ackerboden ausgesät und leicht mit Bodenpartikeln abgedeckt. Unmittelbar nach der Aussaat, auf jeden Fall vor dem Aufkeimen der im Boden befindlichen Samenkörner, wurde der erfindungsgemäß zu verwendende Wirkstoff in Form von Spritzpulvern oder Emulsionskonzentraten auf die vegetationsfreie Bodenoberfläche gleichmäßig aufgespritzt. 4 Wochen nach der Behandlung wurden die Pflanzen abschließend auf Schädigung bzw. Abtötung bonitiert, wobei inzwischen unbehandelt herangewachsene Kontrollpflanzen als Bezug dienten.

Wirksamkeit in % bei Anwendung von 0,5 bis 2 kg/ha Aktivsubstanz im Vorauflauf-Verfahren:

| | kg/ha Aktivsubstanz | | |
|---|---|---|---|
| | 0,5 | 1,0 | 2,0 |
| Nutzpflanzen | | | |
| Baumwolle | 0 | 0 | 10 |
| Sorghum-Hirse | 0 | 0 | 20 |
| Mais | 0 | 0 | 10 |
| Luzerne | 0 | 0 | 10 |
| Erbsen | 0 | 10 | 20 |
| Ackerbohnen | 10 | 30 | 40 |
| Dikotyle Unkräuter | | | |
| Sinapsis arvensis | 98 | 100 | 100 |
| Chenopodium album | 75 | 95 | 98 |
| Chrysanthemum segetum | 100 | 100 | 100 |
| Galinsoga parviflora | 85 | 100 | 100 |
| Centaurea cyanus | 30 | 75 | 98 |
| Stellaria media | 100 | 100 | 100 |
| Polygonum persicaria | 75 | 95 | 100 |
| Galium aparine | 85 | 95 | 98 |
| Amaranthus retroflexus | 100 | 100 | 100 |
| Lamium purpureum | 75 | 100 | 100 |
| Senecio vulgaris | 30 | 75 | 100 |
| Capsella bursa-pastoris | 95 | 98 | 100 |
| Matricaria inodora | 100 | 100 | 100 |

Fortsetzung

| | kg/ha Aktivsubstanz | | |
|---|---|---|---|
| | 0,5 | 1,0 | 2,0 |
| Ungräser | | | |
| Poa annua | 100 | 100 | 100 |
| Alopecurus myosuroides | 95 | 98 | 100 |
| Avena fatua | 95 | 98 | 100 |
| Digitaria sanguinalis | 98 | 100 | 100 |
| Echinochloa crus-galli | 85 | 98 | 100 |

## Beispiel 10

Herbizide Wirksamkeit von 1-Methyl-3-p-chlorphenyl-4-thioparabansäure-5-imid

Der erfindungsgemäß zu verwendende Wirkstoff wird analog Beispiel 9 im Vorauflauf-Verfahren getestet.

Wirksamkeit in % bei Aufwandmengen von 0,5 bis 2 kg/hg Aktivsubstanz im Vorauflaufverfahren:

| | kg/ha Aktivsubstanz | | |
|---|---|---|---|
| | 0,5 | 1,0 | 2,0 |
| Nutzpflanzen | | | |
| Baumwolle | 0 | 0 | 10 |
| Sorghum-Hirse | 0 | 0 | 0 |
| Mais | 0 | 0 | 0 |
| Luzerne | 0 | 10 | 20 |
| Dikotyle Unkräuter | | | |
| Sinapis arvensis | 85 | 100 | 100 |
| Chenopodium album | 80 | 95 | 100 |
| Chrysanthemum segetum | 95 | 100 | 100 |
| Galinsoga parviflora | 98 | 100 | 100 |
| Centaurea cyanus | 70 | 85 | 100 |
| Stellaria media | 90 | 98 | 100 |
| Amaranthus retroflexus | 60 | 100 | 100 |
| Capsella bursa-pastoris | 100 | 100 | 100 |
| Matricaria inodora | 98 | 100 | 100 |
| Solanum nigrum | 80 | 98 | 100 |
| Viola tricolor | 85 | 98 | 100 |

Fortsetzung

| | kg/ha Aktivsubstanz | | |
|---|---|---|---|
| | 0,5 | 1,0 | 2,0 |
| Ungräser | | | |
| Poa annua | 95 | 100 | 100 |
| Alopecurus myosuroides | 85 | 95 | 100 |
| Avena fatua | 50 | 95 | 100 |
| Setaria viridis | 70 | 100 | 100 |
| Apera spica-venti | 100 | 100 | 100 |

Beispiel 11

Herbizide Wirksamkeit von 1-Methyl-3-(3′,4′-dichlorphenyl)-4-thioparabansäure-5-imid

Der erfindungsgemäß zu verwendende Wirkstoff wird analog Beispiel 9 im Vorauflaufverfahren getestet.

Wirksamkeit in % bei Aufwandmengen von 1 bis 4 kg/hg Aktivsubstanz:

| | kg/ha Aktivsubstanz | | |
|---|---|---|---|
| | 1,0 | 2,0 | 4,0 |
| Nutzpflanzen | | | |
| Sorghum-Hirse | 0 | 0 | 0 |
| Mais | 0 | 0 | 0 |
| Luzerne | 0 | 0 | 10 |
| Sommergerste | 0 | 0 | 10 |
| Dikotyle Unkräuter | | | |
| Chenopodium album | 100 | 100 | 100 |
| Chrysanthemum segetum | 98 | 100 | 100 |
| Galinsoga parviflora | 98 | 100 | 100 |
| Stellaria media | 90 | 100 | 100 |
| Amaranthus retroflexus | 60 | 100 | 100 |
| Capsella bursa-pastoris | 100 | 100 | 100 |
| Matricaria inodora | 90 | 100 | 100 |
| Solanum nigrum | 100 | 100 | 100 |
| Viola tricolor | 75 | 100 | 100 |

Fortsetzung

| | kg/ha Aktivsubstanz | | |
|---|---|---|---|
| | 1,0 | 2,0 | 4,0 |
| Ungräser | | | |
| Poa annua | 98 | 100 | 100 |
| Setaria viridis | 85 | 95 | 100 |
| Apera spica-venti | 95 | 100 | 100 |

Vergleichsbeispiel 2

Zum Vergleich wurde das, zu den Harnstoff-Derivaten gehörende Handelsherbizid Afalon mit dem Wirkstoff Linuron [3-(3,4-Dichlorphenyl)-1-methoxy-1-methyl-harnstoff] getestet. Im übrigen wurde wie unter Beispiel 9 beschrieben verfahren.

Wirksamkeit in % bei Anwendung von 0,5 bis 2,0 kg/ha Aktivsubstanz im Vorauflauf-Verfahren:

| | Linuron (Afalon) kg/ha Aktivsubstanz | | |
|---|---|---|---|
| | 0,5 | 1,0 | 2,0 |
| Nutzpflanzen | | | |
| Winter-Weizen | 0 | 10 | 50 |
| Baumwolle | 0 | 0 | 10 |
| Sorghum-Hirse | 0 | 20 | 70 |
| Mais | 0 | 10 | 40 |
| Luzerne | 95 | 100 | 100 |
| Erbsen | 0 | 40 | 70 |
| Ackerbohnen | 10 | 70 | 100 |
| Dikotyle Unkräuter | | | |
| Sinapis arvensis | 98 | 100 | 100 |
| Chenopodium album | 70 | 95 | 98 |
| Chrysanthemum segetum | 85 | 98 | 100 |
| Galinsoga parviflora | 98 | 100 | 100 |
| Centaurea cyanus | 0 | 30 | 70 |
| Stellaria media | 100 | 100 | 100 |
| Polygonum persicaria | 50 | 95 | 100 |
| Galium aparine | 35 | 45 | 50 |
| Amaranthus retroflexus | 100 | 100 | 100 |

Fortsetzung

| | Linuron (Afalon) kg/ha Aktivsubstanz 0,5 | 1,0 | 2,0 |
|---|---|---|---|
| Dikotyle Unkräuter | | | |
| Lamium purpureum | 90 | 98 | 100 |
| Senecio vulgaris | 30 | 80 | 98 |
| Capsella bursa-pastoris | 98 | 98 | 100 |
| Matricaria inodora | 95 | 100 | 100 |
| Ungräser | | | |
| Poa annua | 70 | 80 | 95 |
| Alopecurus myosuroides | 60 | 70 | 85 |
| Avena fatua | 40 | 50 | 80 |
| Digitaria sanguinalis | 70 | 80 | 95 |
| Echinochloa crus-galli | 80 | 95 | 100 |

Der Vergleich der Ergebnisse aus dem Beispiel 9, 10 und 1 mit Vergleichsbeispiel 2 verdeutlicht die verbesserte Nutzpflanzenverträglichkeit der erfindungsgemäß zu verwendenden Wirkstoffe, insbesondere im Bereich der Leguminosen, gegenüber dem Standardprodukt. Darüber hinaus wird die herbizide Wirksamkeit des Vergleichsmittels vor allem gegen die verschiedenen Schadgräser und gegen Klettenlabkraut von den erfindungsgemäß zu verwendenden Substanzen wesentlich übertroffen.

Vergleichsbeispiel 3

In einer weiteren Versuchsreihe wurden Vergleichstests mit dem in Mais-, Baumwoll- und Sojakulturen eingesetzten Handelsprodukt Lasso mit dem Wirkstoff Alachlor [2'-Chlor-2,6-diethyl-N-(methoxy-methyl)-acetanilid] durchgeführt.
Im übrigen wurde wie unter Beispiel 9 beschrieben verfahren.
Wirksamkeit in % bei Anwendung von 0,5 bis 2 kg/ha Aktivsubstanz im Vorauflauf-Verfahren:

| | Lasso (Alachlor) kg/ha Aktivsubstanz 0,5 | 1,0 | 2,0 |
|---|---|---|---|
| Nutzpflanzen | | | |
| Mais | 0 | 10 | 30 |
| Sorghum-Hirse | 85 | 98 | 100 |
| Dikotyle Unkräuter | | | |
| Stellaria media | 40 | 60 | 75 |
| Galium aparine | 0 | 10 | 20 |

Fortsetzung

| | Lasso (Alachlor) kg/ha Aktivsubstanz 0,5 | 1,0 | 2,0 |
|---|---|---|---|
| Dikotyle Unkräuter | | | |
| Centaurea cyanus | 0 | 20 | 40 |
| Chrysanthemum segetum | 0 | 30 | 70 |
| Senecio vulgaris | 20 | 40 | 75 |
| Raphanus raphanistrum | 0 | 10 | 20 |
| Ungräser | | | |
| Avena fatua | 60 | 70 | 85 |
| Alopecurus myosuroides | 50 | 65 | 98 |

Der Vergleich der Ergebnisse aus den Beispielen 9, 10 und 11 mit Vergleichsbeispiel 3 zeigt die wesentlich verbesserte Wirkung der erfindungsgemäß zu verwendenden Verbindungen, vor allem bei der Bekämpfung der Dikotylpflanzen bei vergleichbarer Toleranz gegenüber Mais und sogar entscheidend verbesserter Verträglichkeit gegenüber Sorghum-Hirse.

## Beispiel 12

Herbizide Wirksamkeit von 1-Methyl-3-p-chlorphenyl-4-thioparabansäure-5-imid

Die Einsatzmöglichkeiten des erfindungsgemäß zu verwendenden Wirkstoffs im Nachauflauf-Verfahren (Applikation auf die Blätter der Pflanzen) wurde in einer weiteren Versuchsreihe im Gewächshaus untersucht.

Bei diesem Verfahren wurde der Wirkstoff auf junge Pflanzen gespritzt, die ein bestimmtes Wachstumsstadium erreicht hatten: bei zweikeimblättrigen (dikotylen) Pflanzen war neben den primären Keimblättern das erste, echte Blattpaar entwickelt, bei einkeimblättrigen (monokotylen) Pflanzen waren mindestens zwei Blätter ausgebildet.

Die behandelten Pflanzen wurden das letztemal 14 Tage nach der Spritzung auf Schädigung bzw. Abtötung bonitiert.

Herbizide Wirksamkeit in % bei 1, 2 und 4 kg/ha Aktivsubstanz im Nachauflauf-Verfahren:

| | kg/ha Aktivsubstanz 1 | 2 | 4 |
|---|---|---|---|
| Nutzpflanzen | | | |
| Sommergerste | 0 | 0 | 20 |
| Mais | 0 | 0 | 0 |
| Sorghum-Hirse | 0 | 0 | 20 |
| Luzerne | 0 | 10 | 20 |

Fortsetzung

| | kg/ha Aktivsubstanz | | |
|---|---|---|---|
| | 1 | 2 | 4 |
| Dikotyle Unkräuter | | | |
| Stellaria media | 75 | 98 | 100 |
| Chrysanthemum segetum | 50 | 95 | 100 |
| Galinsoga parviflora | 60 | 98 | 100 |
| Senecio vulgaris | 50 | 70 | 98 |
| Capsella bursa-pastoris | 95 | 100 | 100 |
| Chenopodium album | 90 | 98 | 100 |
| Solanum nigrum | 75 | 90 | 100 |
| Polygonum persicaria | 60 | 85 | 98 |
| Ungräser | | | |
| Poa annua | 70 | 98 | 100 |
| Apera spica-venti | 70 | 95 | 100 |

Beispiel 13

Herbizide Wirksamkeit von 1-Methyl-3-(3',4'-dichlorphenyl)-4-thioparabansäure-5-imid im Nachauflaufverfahren

Es wurde analog Beispiel 12 bei Aufwandmengen von 0,5 bis 2 kg/ha Aktivsubstanz verfahren. Herbizide Wirksamkeit bei 0,5 bis 2 kg/ha Aktivsubstanz im Nachauflaufverfahren:

| | 0,5 | 1 | 2 |
|---|---|---|---|
| Nutzpflanzen | | | |
| Sommergerste | 0 | 0 | 0 |
| Mais | 0 | 0 | 0 |
| Sorghum-Hirse | 0 | 0 | 0 |
| Luzerne | 0 | 0 | 20 |
| Dikotyle Unkräuter | | | |
| Stellaria media | 80 | 95 | 100 |
| Chrysanthemum segetum | 90 | 98 | 100 |
| Galinsoga parviflora | 85 | 90 | 98 |
| Centauria cyanus | 60 | 95 | 98 |

Fortsetzung

| | 0,5 | 1 | 2 |
|---|---|---|---|
| Dikotyle Unkräuter | | | |
| Capsella bursa-pastoris | 95 | 100 | 100 |
| Chenopodium album | 98 | 100 | 100 |
| Solanum nigrum | 75 | 80 | 100 |
| Polygonum persicaria | 95 | 98 | 100 |
| Amaranthus retroflexus | 95 | 100 | 100 |
| Galium aparine | 70 | 85 | 95 |

**Patentanspruch**

Verwendung einer oder mehrerer Verbindungen der allgemeinen Formel I

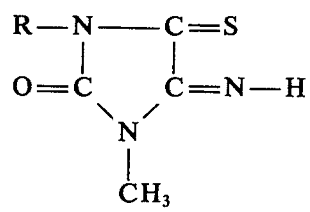

(I)

als herbizide Wirkstoffe,
in der R entweder die Naphthylgruppe oder eine einfach oder mehrfach substituierte Phenylgruppe bedeutet, wobei als Substituenten in Betracht kommen:
Methyl, Ethyl, Propyl, Isopropyl, Methoxy, Ethoxy, Propoxy, Methylmercapto, Ethylmercapto, Propylmercapto, Methoxycarbonyl, Ethoxycarbonyl, Propoxycarbonyl, Methylsulfonato, Ethylsulfonato, Trifluormethyl, Halogen, die Nitro- und die Cyanogruppe.

**Claim**

Use of one or more compounds of the general formula I

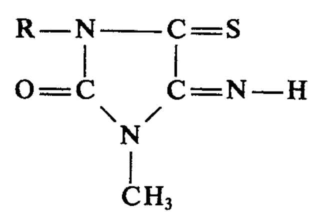

(I)

as herbicidally active substances,
in which R represents either a naphthyl group or a monosubstituted or polysubstituted phenyl group in which the substituents are methyl, ethyl, propyl, isopropyl, methoxy, ethoxy, propoxy, methylthio, ethylthio, propylthio, methoxycarbonyl, ethoxycarbonyl, propoxycarbonyl, methylsulphonato, ethylsulphonato, trifluoromethyl, halogen, nitro and cyano groups.

**Revendication**

Utilisation comme matières actives herbicides d'un ou plusieurs composés répondant à la formule générale I:

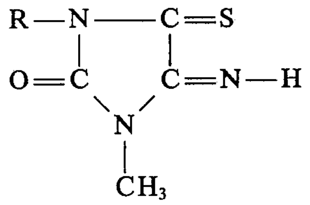

(I)

dans laquelle R représente soit le groupe naphtyle, soit un groupe phényle mono- ou polysubstitué, les substituants envisagés étant les groupes:
méthyle, éthyle, propyle, isopropyle, méthoxy, éthoxy, propoxy, méthylthio, éthylthio, propylthio, méthoxycarbonyle, éthoxycarbonyle, propoxycarbonyle, méthylsulfonato, éthylsulfonato, trifluorométhyle, halogène, nitro et cyano.